# EUROPEAN PATENT APPLICATION

(11) **EP 1 671 655 A1**
(43) Date of publication of application: **21.06.2006**
(21) Application number: 04772726.8
(22) Date of filing: 27.08.2004
(51) Int. Cl.: A61K 48/00, A61K 38/16, A61K 31/7088, A61P 17/00, A61P 17/02, A61P 17/06, A61K 9/08

(54) **GENE THERAPY FOR SKIN DISEASES USING NEEDLE FREE SYRINGE**

(30) Priority: 29.08.2003 JP 2003307713
(71) Applicant: AnGes MG, Inc., Ibaraki-shi, Osaka 567-0085 (JP)
(72) Inventor: KUNUGIZA, Yasuo, Minoo-shi, Osaka 5620027 (JP); TOMITA, Naruya, Osaka-shi, Osaka 5430051 (JP); YOSHIKAWA, Hideki, Toyonaka-shi, Osaka 5600004 (JP); TANIYAMA, Yoshiaki, Suita-shi, Osaka 5650821 (JP); TAMAI, Katsuto, Ibaraki-shi, Osaka 5670046 (JP); MORISHITA, Ryuichi, Suita-shi, Osaka 5650851 (JP)
(74) Representative: Grünecker, Kinkeldey, Stockmair & Schwanhäusser Anwaltssozietät
(86) International application number: PCT/JP2004/012777
(87) International publication number: WO 2005/021045

(57) **Abstract**

The present invention provides highly useful clinical methods for treating skin disorders, particularly intractable skin disorders. Specifically, the methods comprise injecting/subcutaneously introducing a 10 µg to 10 mg dose of a polynucleotide, such as a DNA, oligonucleotide, RNA, siRNA, and antisense, around a lesion associated with a skin disorder, such as a wound, cutaneous ulcer, or psoriasis, using a needleless syringe that injects a pharmaceutical liquid by using gas pressure or the elastic force of an elastic member to drive a piston.

SEQUENCE LISTING

<110> ANGES MG, INC.

<120> GENE THERAPY FOR SKIN DISEASES USING NEEDLE FREE SYRINGE

<130> MED-A0402P

<150> JP 2003-307713
<151> 2003-08-29

<160> 2

<210> 1
<211> 20
<212> DNA
<213> Artificial Sequence

<220>
<223> Description of Artificial Sequence Synthetic DNA

<400> 1
ccttgaaggg atttccctcc                                                20

<210> 2

<211> 20

<212> DNA

<213> Artificial Sequence

<220>

<223> Description of Artificial Sequence Synthetic DNA

<400> 2

ttgccgtacc tgacttagcc                                                    20

## Description

### Technical Field

The present invention relates to methods for treating skin disorders by subcutaneously introducing polynucleotides using needleless syringes.

### Background Art

There are a wide variety of skin disorders, including, for example, wounds, cutaneous ulcers, atopic dermatitis, bedsores, bum injuries, perniosis, optic hyperesthesia, psoriasis, palmoplantar pustulosis, eczema, and scleriasis. Many skin disorders are intractable.

Of these, chronic (intractable) wounds are defined specifically as "wounds that fail to heal in an orderly and timely manner to produce anatomical and functional integrity, or wounds that fail to proceed through anatomical and functional repair processes". Such wounds do not respond, in shape or appearance, to any form of appropriate proactive treatment, and exhibit no signs of healing even after two to four weeks. Chronic (intractable) wounds are an intractable skin disorder.

Cutaneous ulcers, another intractable skin disorder, are caused by cell necrosis resulting from sustained tissue ischemia, whose cause is vascular, and lymphatic congestion due to prolonged pressure, friction, bedsores, or peripheral circulatory failure (vasoocclusive lesions and the like).

Psoriasis has several clinical disease types. The common type is psoriasis vulgaris, which is an intractable chronic inflammatory hyperkeratotic disease, accompanied by (1) thickening and keratinization of the epidermis, and (2) infiltration of inflammatory cells into the epidermis/dermis.

Previously, pharmaceutical agents, such as adrenocortical hormones, immunosuppressants, vitamin A acid, active vitamin D3, antiviral agents, interferon, and prostaglandin, have been used in the treatment of such skin diseases. However, none of these agents are satisfactory in terms of their effectiveness, safety, prevention of recurrence, and such.

US 2002/064876 and Japanese Patent Kohyo Publication No. (JP-A) 2003-502350 (unexamined Japanese national phase publication corresponding to a non-Japanese international publication) are incorporated herein by reference.

### Disclosure of the Invention

The present invention addresses the absence of a highly useful clinical method for treating skin disorders, particularly intractable skin disorders.

Specifically, the present invention provides the following methods for treating skin disorders:
[1] a method for treating a skin disorder comprising introducing a polynucleotide subcutaneously using a needleless syringe;
[2] a method for treating a skin disorder comprising injecting/subcutaneously introducing a polynucleotide around diseased skin using a needleless syringe;
[3] the method of [1] or [2], wherein the polynucleotide is selected from a DNA, oligonucleotide, RNA, siRNA, and antisense;
[4] the method of any one of [1] to [3], comprising injecting/subcutaneously introducing 10 µg to 10 mg of the polynucleotide per dose in portions to multiple sites around the diseased skin;
[5] the method of any one of [1] to [4], wherein the needleless syringe injects a pharmaceutical liquid by using a gas pressure or an elastic force of an elastic member to drive a piston;
[6] the method of [5], wherein the gas is helium, nitrogen, or air, and the elastic member is a spring;
[7] the method of any one of [1] to [6], wherein the polynucleotide is hepatocyte growth factor (HGF) gene and/or prostacyclin synthetase (PGIS) gene;
[8] the method of any one of [1] to [7], wherein the oligonucleotide is an NF-κB decoy oligonucleotide comprising the sequence of SEQ ID NO: 1 or 2;
[9] the method of any one of [1] to [8], wherein the skin disorder is a wound, cutaneous ulcer, or psoriasis;
[10] the method of any one of [1] to [9], wherein the wound is a post-surgical wound or a wound caused by an injury or accident;
[11] the method of any one of [1] to [10], wherein the cutaneous ulcer is an intractable cutaneous ulcer;
[12] the method of any one of [1] to [11], wherein the intractable cutaneous ulcer is a diabetic ulcer, bedsore (pressure ulcer), or ulcer associated with venous or arterial insufficiency;
[13] a method for treating a wound or cutaneous ulcer, comprising injecting/subcutaneously introducing an HGF gene and/or PGIS gene around diseased skin using a needleless syringe;
[14] the method of [13], comprising injecting/subcutaneously introducing the HGF gene and PGIS gene around the diseased skin using a needleless syringe;
[15] a method for treating psoriasis, comprising injecting/subcutaneously introducing an NF-κB decoy oligonucleotide around diseased skin using a needleless syringe;
[16] an agent for treating, ameliorating, or preventing a skin disorder, comprising a polynucleotide as an active ingredient, wherein the agent is introduced subcutaneously using a needleless syringe;
[17] an agent for treating, ameliorating, or preventing a skin disorder, comprising a polynucleotide as an active ingredient, wherein the agent is injected/subcutaneously introduced around diseased skin using a needleless syringe;
[18] the agent of [16] or [17], wherein the polynucleotide is selected from a DNA, oligonucleotide, RNA, siRNA, and antisense;
[19] the agent of any one of [16] to [18], comprising 10 µg to 10 mg of the polynucleotide per dose as an active ingredient, wherein the agent is injected/subcutaneously introduced in portions to multiple sites around the diseased skin;
[20] the agent of any one of [16] to [19], wherein the needleless syringe injects a pharmaceutical liquid by using a gas pressure or an elastic force of an elastic member to drive a piston;
[21] the agent of [20], wherein the gas is helium, nitrogen, or air, and the elastic member is a spring;
[22] the agent of any one of [16] to [21], wherein the polynucleotide is an HGF gene and/or PGIS gene;
[23] the agent of any one of [16] to [22], wherein the oligonucleotide is an NF-κB decoy oligonucleotide comprising the sequence of SEQ ID NO: 1 or 2;
[24] the agent of any one of [16] to [23], wherein the skin disorder is a wound, cutaneous ulcer, or psoriasis;
[25] the agent of any one of [16] to [24], wherein the wound is a post-surgical wound or a wound caused by an injury or accident;
[26] the agent of any one of [16] to [25], wherein the cutaneous ulcer is an intractable cutaneous ulcer;
[27] the agent of any one of [16] to [26], wherein the intractable cutaneous ulcer is a diabetic ulcer, bedsore (pressure ulcer), or ulcer associated with venous or arterial insufficiency;
[28] an agent for treating, ameliorating, or preventing a wound or cutaneous ulcer, comprising an HGF gene and/or PGIS gene as an active ingredient, wherein the agent is injected/subcutaneously introduced around diseased skin using a needleless syringe;
[29] the agent of [28], comprising an HGF gene and a PGIS gene as active ingredients, wherein the agent is injected/subcutaneously introduced around diseased skin using a needleless syringe;
[30] an agent for treating, ameliorating, or preventing psoriasis, comprising an NF-κB decoy oligonucleotide as an active ingredient, wherein the agent is injected/subcutaneously introduced around diseased skin using a needleless syringe;
[31] use of a polynucleotide for preparing an agent for treating, ameliorating, or preventing a skin disorder, wherein the agent is introduced subcutaneously using a needleless syringe;
[32] use of a polynucleotide for preparing an agent for treating, ameliorating, or preventing a skin disease, wherein the agent is injected/subcutaneously introduced around diseased skin using a needleless syringe;
[33] the use of [31] or [32], wherein the polynucleotide is any one selected from a DNA, oligonucleotide, RNA, siRNA, and antisense;
[34] the use of any one of [31] to [33], wherein 10 µg to 10 mg of the polynucleotide per dose is injected/subcutaneously introduced in portions to multiple sites around the diseased skin;
[35] the use of any one of [31] to [34], wherein the needleless syringe injects the pharmaceutical liquid by using a gas pressure or an elastic force of an elastic member to drive a piston;
[36] the use of [35], wherein the gas is helium, nitrogen, or air, and the elastic member is a spring;
[37] the use of any one of [31] to [36], wherein the polynucleotide is an HGF gene and/or PGIS gene;
[38] the use of any one of [31] to [37], wherein the oligonucleotide is an NF-κB decoy oligonucleotide that comprises the sequence of SEQ ID NO: 1 or 2;
[39] the use of any one of [31] to [38], wherein the skin disorder is a wound, cutaneous ulcer, or psoriasis;
[40] the use of any one of [31] to [39], wherein the wound is a post-surgical wound or a wound caused by an injury or accident;
[41] the use of any one of [31] to [40], wherein the cutaneous ulcer is an intractable cutaneous ulcer;
[42] the use of any one of [31] to [41], wherein the intractable cutaneous ulcer is a diabetic ulcer, bedsore (pressure ulcer), or ulcer associated with venous or arterial insufficiency;
[43] use of an HGF gene and/or PGIS gene for preparing an agent for treating, ameliorating, or preventing a wound or cutaneous ulcer, wherein the agent is injected/subcutaneously introduced around diseased skin using a needleless syringe;
[44] the use of [43] of the HGF gene and PGIS gene for preparing an agent for treating, ameliorating, or preventing, wherein the agent is injected/subcutaneously introduced around diseased skin using a needleless syringe; and
[45] use of an NF-κB decoy oligonucleotide for preparing an agent for treating, ameliorating, or preventing psoriasis, wherein the agent is injected/subcutaneously introduced around diseased skin using a needleless syringe.

In the context of the present invention, the phrase "needleless syringe" refers to a medical apparatus for administering pharmaceutical ingredients subcutaneously, more preferably into subcutaneous cells, by using gas pressure or the elastic force of an elastic member to drive a piston and thus inject a pharmaceutical liquid into the skin without using an injection needle.

Examples of commercially available needleless syringes include ShimaJET™ (Shimadzu Co.), Medi-Jector Vision™ (Elitemedical), PenJet™ (PenJet), and such.

Unlike conventional needle syringes, needleless syringes are advantageous in that they can avoid pain and the risk of infection, for example.

The polynucleotides of the present invention specifically include, for example, DNAs, oligonucleotides, RNAs, siRNAs, and antisenses.

These polynucleotides may be naked or inserted into various vectors or plasmids.

The polynucleotides of the present invention include, without limitation, any known polynucleotide. Examples of preferred polynucleotides include angiogenesis factor genes, prostacyclin synthetase (PGIS) gene, nitric oxide synthase (NOs) gene, and decoy oligonucleotides for transcriptional factors.

Specific examples of angiogenesis factor genes include hepatocyte growth factor (HGF) gene, vascular endothelial growth factor (VEGF) gene, epidermal growth factor (EGF) gene, and fibroblast growth factor (FGF) gene. Of these, the HGF gene is more preferred. The sequence of the HGF gene has been previously described, for example in Japanese Patent No. 2577091.

The term "PGIS gene" refers to the gene encoding an enzyme involved in the process of forming prostaglandin I2 (PGI2) from prostaglandin H2 (PGH2). Its specific sequence has been previously described, for example in Japanese Patent Saikohyo Publication No. (JP-A) 95/030013 (unexamined Japanese national phase publication corresponding to a Japanese international publication).

Specific examples of decoy oligonucleotides for transcriptional factors include NF-κB decoy oligonucleotides, E2F decoy oligonucleotides, AP-1 decoy oligonucleotides, Ets decoy oligonucleotides, STAT-1 decoy oligonucleotides, STAT-6 decoy oligonucleotides and GATA-3 decoy oligonucleotides. Of these, NF-κB decoy oligonucleotides are more preferred.

Specific examples of NF-κB decoy oligonucleotides include sequences comprising GGGRA(C,T)TYYA(C,T)C (R and Y represent purine and pyrimidine nucleotides, respectively), and more specifically, oligonucleotides that comprise the sequence shown in SEQ ID NOs: 1 or 2 of the instant Sequence Listing, for example.

The types of skin disorders addressed by the present invention are not limited. In particular, intractable skin disorders are preferred. Specifically, such disorders include, for example, wounds, cutaneous ulcers, and psoriasis.

Examples of wounds specifically include, but are not limited to, post-surgical wounds and wounds caused by injury or accident.

There is no limitation as to the type of cutaneous ulcer. Examples of preferred cutaneous ulcers include intractable cutaneous ulcers, more preferably diabetic ulcers, bedsores (pressure ulcers) and ulcers associated with venous or arterial insufficiencies.

The most preferred embodiments of the present invention include the following combinations:
(1) methods for treating wounds or cutaneous ulcers, which comprise using a needleless syringe to inject/subcutaneously introduce HGF gene at multiple sites around the diseased area;
(2) methods for treating wounds or cutaneous ulcers, which comprise using a needleless syringe to inject/subcutaneously introduce PGIS gene at multiple sites around the diseased area;
(3) methods for treating wounds or cutaneous ulcers, which comprise using a needleless syringe to inject/subcutaneously introduce HGF gene and PGIS gene at multiple sites around the diseased area; and
(4) methods for treating psoriasis, which comprise using a needleless syringe to inject/subcutaneously introduce NF-κB decoy oligonucleotide at multiple sites around the diseased area.

In the context of the present invention, the doses of polynucleotides are not limited, and depend on the type and severity of the disorder, the location and size of the diseased site, the patient's age and sex, complications, concomitant drugs, and other factors. In general, doses of 10 µg to 10 mg of polynucleotide are preferably injected/introduced subcutaneously in portions to multiple sites around the diseased skin.

All prior-art documents cited herein are incorporated herein by reference.

### Brief Description of the Drawings

Fig. 1 is a set of photographs demonstrating that expression of Yellow Fluorescence Protein (Venus) was detected only in epidermal tissues.
Fig. 2 is a set of photographs demonstrating that LacZ expression was detected only in epidermal tissues.
Fig. 3 shows that the ShimaJET-injected group exhibited about 100 times more luciferase activity than the needle-injected group, and thus achieved greater introduction efficiency.
Fig. 4 shows that a wound healing-accelerating effect was observed in the HGF and/or PGIS injected groups on days 4 and 6, and that an enhanced effect was found in the simultaneously injected group (group 5).
Fig. 5 is a set of photographs demonstrating that both HGF and PGIS were effective in enhancing blood flow around the wound.
Fig. 6 is a set of photographs demonstrating that both the HGF and PGIS genes were expressed in wounded epidermal tissue.
Fig. 7 shows that increased levels of HGF protein were found.

### Best Mode for Carrying out the Invention

Herein below, the present invention is more specifically described using Examples; however, the invention should not to be construed as being limited thereto.

### [Example 1]

### Verification of introduction sites

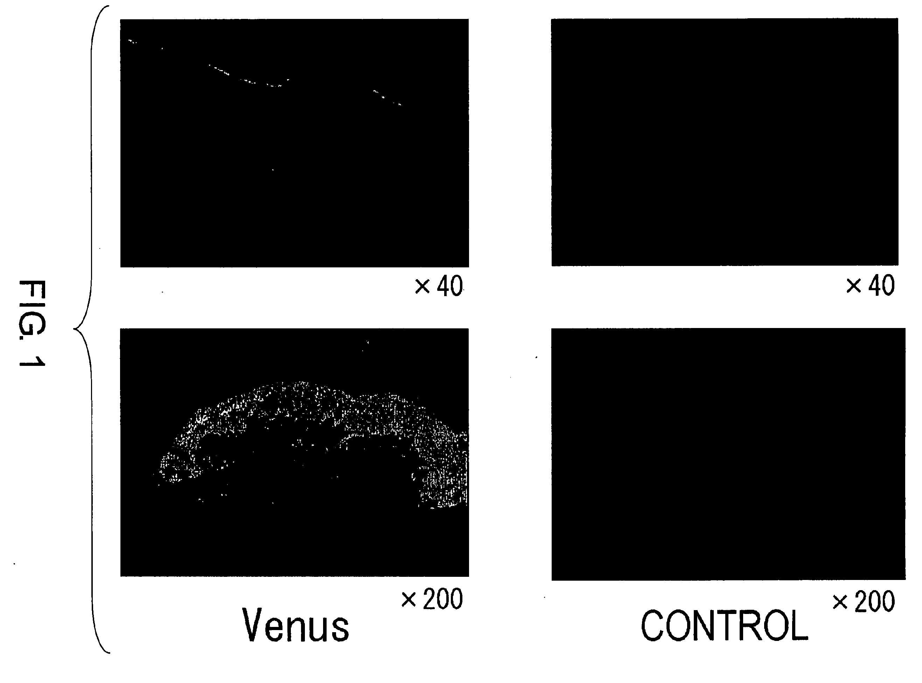

Verification of sites introduced with Yellow Fluorescence Protein (Venus) plasmid (Venus/pCS2), by non-needle injection into rat skin (see Fig. 1).

### Methods:

(1) The dorsal skin of rats was shaved and then depilated using Kanebo epilat hair remover cream. Then, 100 µg/100 µl of Yellow Fluorescence Protein (Venus)/pCS2 was injected using ShimaJET. For comparison, plasmid (pCS2) without Venus was used as a control.
(2) The rats were sacrificed after 24 hours, and the skin at the injection site was collected.
(3) The tissues were placed into OCT compound and frozen rapidly in liquid nitrogen, then sectioned and observed under a fluorescence microscope.

### Results:

Expression of Yellow Fluorescence Protein (Venus) was detected only in epidermal tissues.

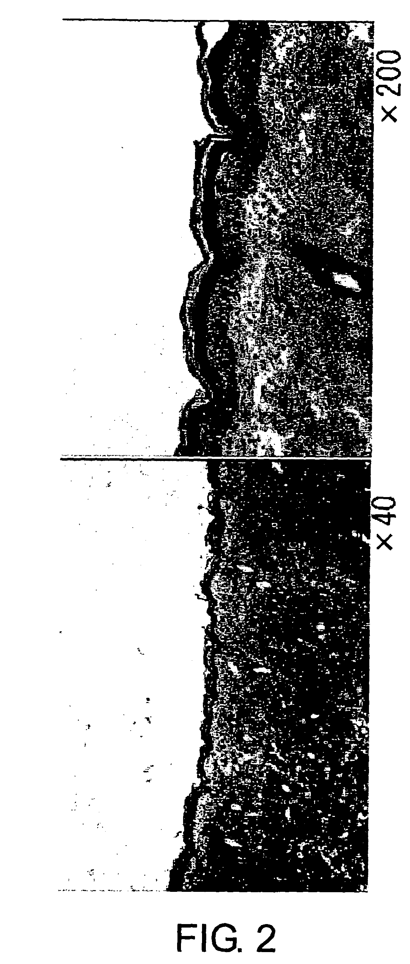

Verification of sites introduced with lacZ plasmid (pcLacZ) by non-needle injection into rat skin (see Fig. 2).

### Methods:

(1) The dorsal skin of rats was shaved and depilated. Then, 200 µg/200 µl of pcLacZ was injected using ShimaJET.
(2) The rats were sacrificed after 24 hours, and the skin at the injection site was collected.
(3) The tissues were placed into OCT compound, frozen rapidly in liquid nitrogen, and then sectioned. The sections were fixed with 1% glutaraldehyde, stained using a β-gal staining solution, and observed under a microscope.

### Results:

Expression of LacZ was detected only in epidermal tissues.

### [Example 2]

### Evaluation of introduction efficiency

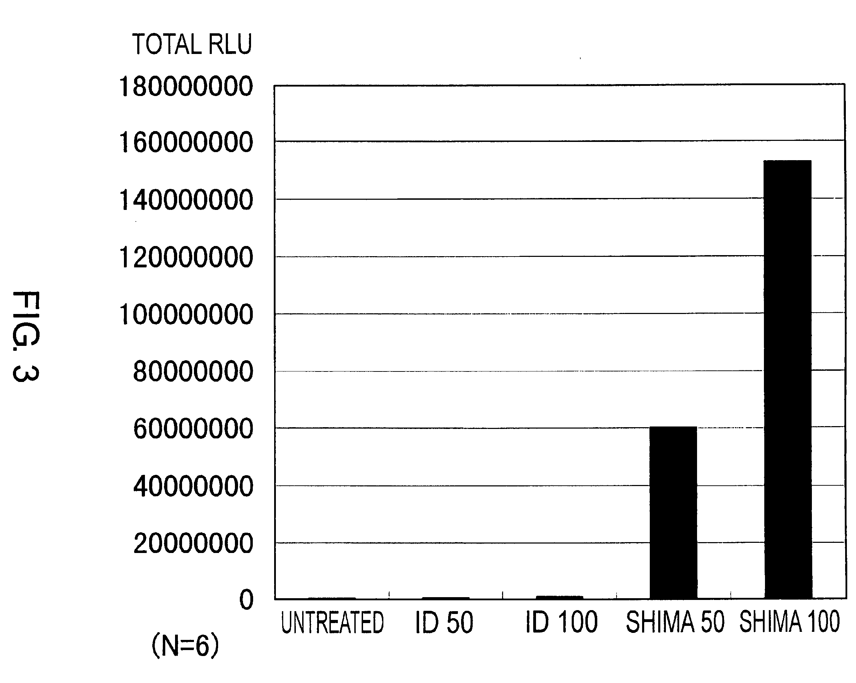

Luciferase activity assay (see Fig. 3)

### Method:

(1) The dorsal skin of rats was shaved and depilated. Then, 50 µg/50 µl or 100 µg/100 µl of luciferase plasmid (pGL3 luc) was injected into the rat's skin using ShimaJET. For comparison, rats were intradermally needle-injected with an equal amount of the plasmid using a 26G syringe.
(2) The rats were sacrificed after 24 hours, and about two square centimeters of the skin centered on the injection site was collected. As a control, skin was collected from untreated rats.
(3) 1 ml of Luciferase Lysis Buffer (Promega) was added, and the skin was cut with scissors into the smallest pieces as possible.
(4) The skin was frozen rapidly at -80°C for ten minutes, and then thawed at room temperature. This treatment was repeated twice.
(5) After centrifugation at 5000 rpm for ten minutes, the supernatant was collected.
(6) Luciferase activity was assayed (Berthold LB9507).
   Fig. 3 shows total luciferase activity after each treatment. In Fig. 3, "ID 50" and "ID 100" indicate that the rats were needle-injected intradermally with 50 µg/50 µl and 100 µg/100 µl of pGL3 luc plasmid, respectively, and "Shima 50" and "Shima 100" indicate that 50 µg/50 µl and 100 µg/100 µl of pGL3 luc plasmid were injected into the rat skin using ShimaJET, respectively.

### Results:

The ShimaJET-injected group exhibited about 100 times more luciferase activity than the needle-injected group, confirming a high introduction efficiency.

### [Example 3]

### Assessment of wound healing effect

### Preparation of a rat model of impaired wound healing:

A rat model of impaired wound healing was prepared by administering water-soluble prednisolone (prednisolone sodium succinate: Pz, Shionogi & CO., LTD) as a steroid to rats. Steroid administration to the steroid-administration rat model causes disorders in wound tensile strength, epithelialization, angiogenesis, wound contraction, and so on, resulting in retardation of wound healing.
(1) 7-week-old male Wister rats were needle-injected intramuscularly with water-soluble prednisolone (Prednisolone Sodium Succinate, Shionogi & CO., LTD) at a dose of 30 mg/kg. After three days (on the day of wound creation), water-soluble prednisolone was again needle-injected intramuscularly at a dose of 30 mg/kg. The control group was needle-injected intramuscularly with PBS.
(2) After shaving and depilating the dorsal skin of rats, wounds were created by removing entire layers of skin in a circle of diameter 1.6 cm (an area of about 2 cm²). *Plasmid introduction:*
(3) Plasmid (HGF, PGIS, or HGF+PGIS) was injected at five sites around the wound using ShimaJET. In a single injection of each plasmid, the amount of plasmid and the volume of solution used were 100 µg and 100 µl respectively.

PBS was needle-injected as a control (n=6 in each group).

HGF plasmid: CAS registry No. [627861-07-8]

PGIS plasmid: the cDNA of SEQ ID NO: 11 shown in WO 95/30013 was inserted into pVAX1 (Invitrogen).

| | Intramuscular needle-injection | ShimaJET (day 0) | ShimaJET (day 2) |
|---|---|---|---|
| Group 1 | PBS | PBS | |
| Group 2 | PZ | PBS | |
| Group 3 | PZ | HGF (100 µg x 5) | |
| Group 4 | PZ | PGIS (100 µg x 5) | PGIS (100 µg x 5) |
| Group 5 | PZ | HGF (100 µg x 5) + PGIS (100 µg x 5) | |

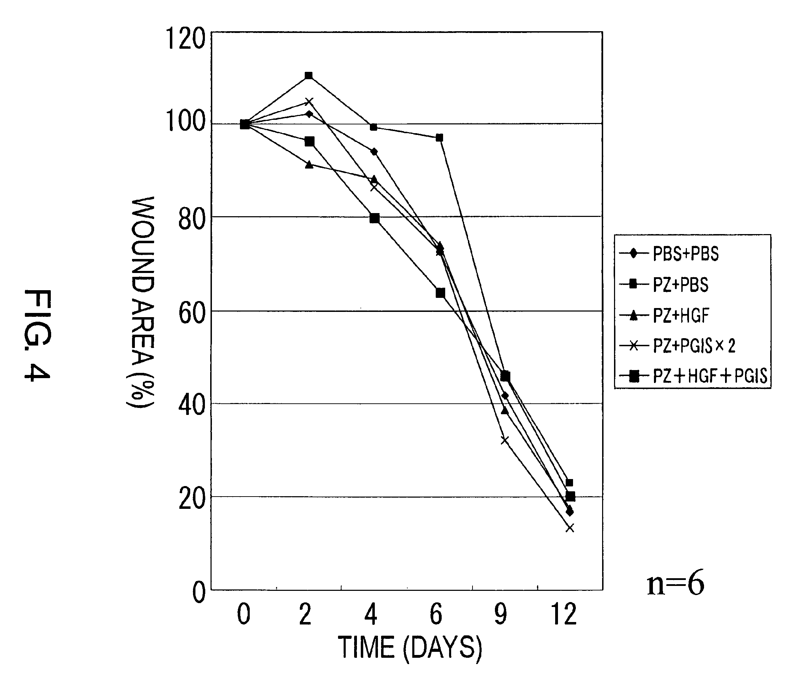

### Measurement of wound area (see Fig. 4 and the table shown below):

(4) On days 0, 2, 4, 6, 9, and 12 of wound creation, the wounds were traced onto tracing paper. These images were then scanned with a scanner and their areas were computed using NIH image 1.61. The area on day 0 was taken as 100, and relative areas on subsequent days were then computed.

### Results:

On days 4 and 6, a wound healing-accelerating effect was obtained in the HGF and/or PGIS non-needle injected group. The effect was found to be enhanced in the group simultaneously injected (group 5).

| | | | | | | Wound area (%) | |
|---|---|---|---|---|---|---|---|
| | | 0 | 2 | 4 | 6 | 9 | 12 (days) |
| Group 1 | PBS+PBS | 100 | 102.04 | 94.04 | 72.54 | 41.68 | 16.72 |
| Group 2 | PZ+PBS | 100 | 110.5 | 99.2 | 96.85 | 46.5 | 23 |
| Group 3 | PZ+HGF | 100 | 91.3 | 88.1 | 74 | 38.66 | 17.5 |
| Group 4 | PZ+PGIS x 2 | 100 | 104.8 | 86.45 | 72.6 | 31.95 | 13.45 |
| Group 5 | PZ+HGF+PGIS | 100 | 96.4 | 79.725 | 64.1 | 46.1 | 20.1 |

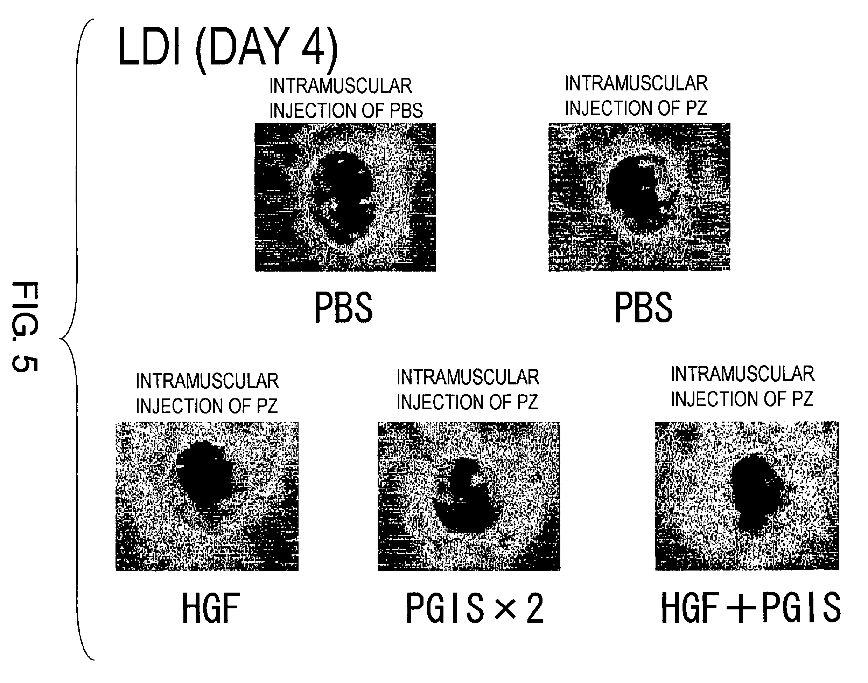

### Measurement of blood flow (see Fig. 5):

(5) Four days after wound creation, blood flow around the wounds in the dorsal areas of the rats was monitored using Laser Doppler Imager (Moor).
Results: Both HGF and PGIS were found to be effective in enhancing blood flow around the wounds.

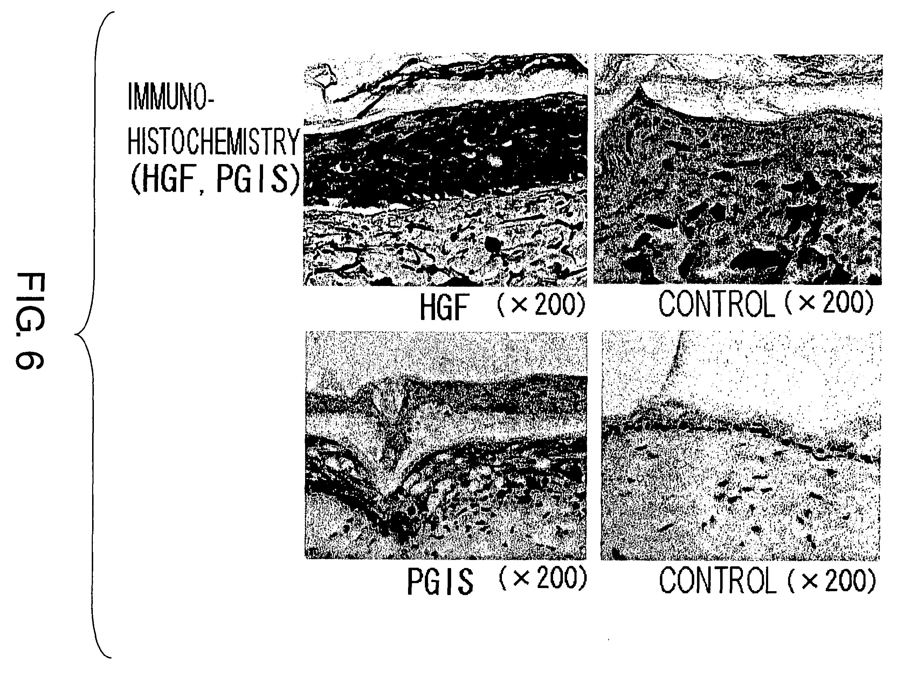

### Confirmation of expression of HGF and PGIS genes (see Fig. 6):

Skin was collected from wound sites and immunostained for HGF and PGIS using the following procedures:

### Immunostaining for HGF:

The collected skin samples were fixed in paraffin and then sectioned. The sections were deparaffinized by three treatments with 100% xylene for five minutes, two treatments with 100% alcohol for five minutes, a single treatment with 99% alcohol for five minutes, a single treatment with 90% alcohol for five minutes, and a single treatment with 75% alcohol for five minutes, and then washed with water for ten minutes. Antigen retrieval was achieved at 95°C for 15 minutes. The sections were treated with 80% methanol/0.6% hydrogen peroxide and 3% hydrogen peroxide, and then blocked with normal goat serum for 30 minutes. Ten-fold diluted primary antibody (rabbit anti-human HGFb (H495) polyclonal antibody, 18134, Immuno-Biological Laboratories Co., Ltd.) was added, and the sections were then incubated at 4°C overnight. After washing off the primary antibody, a secondary antibody (anti-rabbit antibody, Vectastain Elite ABC kit, biotinylated antibody) was added, and the sections were incubated at room temperature for 30 minutes. After washing off the secondary antibody, Vectastain Elite ABC reagent was added, and the sections were incubated for 30 minutes. The sections were stained with DAB (Funakoshi), and then counterstained with hematoxylin/eosin. The sections were dehydrated, mounted, and then observed under a microscope.

### Results:

HGF expression was detected in epidermal tissues.

### Immunostaining for PGIS:

Frozen sections were prepared from the collected skin samples and dried overnight in a freezer, then fixed with cold acetone (-20°C) for 15 minutes. After blocking avidin and biotin, the sections were treated with 80% methanol/0.6% hydrogen peroxide and 3% hydrogen peroxide. A complex antibody (mixture of a primary antibody (rabbit anti-PGIS C-terminal peptide polyclonal antibody (1000-fold diluted)), secondary antibody (biotin-labeled goat anti-rabbit antibody (DAKO E0432)(300-fold diluted)) and normal rabbit serum) was added, and then the sections were incubated at 4°C overnight. After washing off the antibodies, the sections were reacted with peroxidase-labeled streptavidin (DAKO E1016) at room temperature for 30 minutes, stained with DAB (Funakoshi), and then counterstained with hematoxylin/eosin. The sections were then dehydrated, mounted, and observed under a microscope.

### Results:

PGIS expression was detected in epidermal tissues.

### [Example 4]

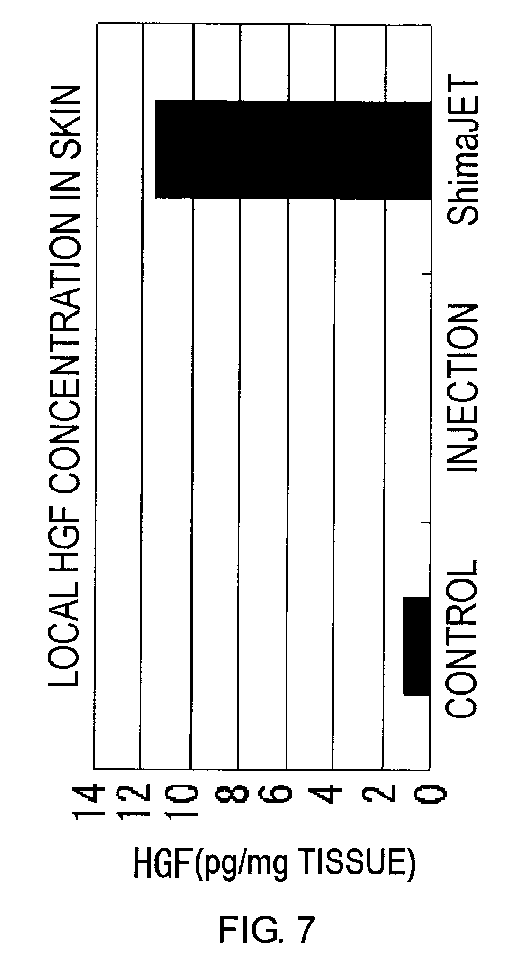

### Quantitation of HGF protein (see Fig. 7)

### Method:

(1) After shaving and depilating the dorsal skin of rats, HGF plasmid was injected using ShimaJET. After 24 hours, the rats were sacrificed and tissues (about 40 mg) were collected.
(2) The tissues were washed with 800 µl of PBS, and homogenized with ten times the volume of extraction buffer (extraction buffer: 20 mM Tris-HCl buffer (pH 7.5); 2 M NaCl; 0.1% Tween 80; 1 mM EDTA; 1 mM PMSF).
(3) The tissues were then centrifuged at 15000 rpm for 30 minutes at 4°C.
(4) The supernatants were collected and assayed using ELISA (Biosource).

### Results:

The level of HGF protein was found to be increased.

### Industrial Applicability

The present invention provides methods for treating skin disorders, more particularly, methods for treating intractable skin disorders for which, to date, there is no highly useful clinical method for treatment available.

## Claims

1. A method for treating a skin disorder comprising introducing a polynucleotide subcutaneously using a needleless syringe.

2. A method for treating a skin disorder comprising injecting/subcutaneously introducing a polynucleotide around diseased skin using a needleless syringe.

3. The method of claim 1 or 2, wherein the polynucleotide is selected from a DNA, oligonucleotide, RNA, siRNA, and antisense.

4. The method of any one of claims 1 to 3, comprising injecting/subcutaneously introducing 10 µg to 10 mg of the polynucleotide per dose in portions to multiple sites around the diseased skin.

5. The method of any one of claims 1 to 4, wherein the needleless syringe injects a pharmaceutical liquid by using a gas pressure or an elastic force of an elastic member to drive a piston.

6. The method of claim 5, wherein the gas is helium, nitrogen, or air, and the elastic member is a spring.

7. The method of any one of claims 1 to 6, wherein the polynucleotide is hepatocyte growth factor (HGF) gene and/or prostacyclin synthetase (PGIS) gene.

8. The method of any one of claims 1 to 7, wherein the oligonucleotide is an NF-κB decoy oligonucleotide comprising the sequence of SEQ ID NO: 1 or 2.

9. The method of any one of claims 1 to 8, wherein the skin disorder is a wound, cutaneous ulcer, or psoriasis.

10. The method of any one of claims 1 to 9, wherein the wound is a post-surgical wound or a wound caused by an injury or accident.

11. The method of any one of claims 1 to 10, wherein the cutaneous ulcer is an intractable cutaneous ulcer.

12. The method of any one of claims 1 to 11, wherein the intractable cutaneous ulcer is a diabetic ulcer, bedsore (pressure ulcer), or ulcer associated with venous or arterial insufficiency.

13. A method for treating a wound or cutaneous ulcer, comprising injecting/subcutaneously introducing an HGF gene and/or PGIS gene around diseased skin using a needleless syringe.

14. The method of claim 13, comprising injecting/subcutaneously introducing the HGF gene and PGIS gene around the diseased skin using a needleless syringe.

15. A method for treating psoriasis, comprising injecting/subcutaneously introducing an NF-κB decoy oligonucleotide around diseased skin using a needleless syringe.

16. An agent for treating, ameliorating, or preventing a skin disorder, comprising a polynucleotide as an active ingredient, wherein the agent is introduced subcutaneously using a needleless syringe.

17. An agent for treating, ameliorating, or preventing a skin disorder, comprising a polynucleotide as an active ingredient, wherein the agent is injected/subcutaneously introduced around diseased skin using a needleless syringe.

18. The agent of claim 16 or 17, wherein the polynucleotide is selected from a DNA, oligonucleotide, RNA, siRNA, and antisense.

19. The agent of any one of claims 16 to 18, comprising 10 µg to 10 mg of the polynucleotide per dose as an active ingredient, wherein the agent is injected/subcutaneously introduced in portions to multiple sites around the diseased skin.

20. The agent of any one of claims 16 to 19, wherein the needleless syringe injects a pharmaceutical liquid by using a gas pressure or an elastic force of an elastic member to drive a piston.

21. The agent of claim 20, wherein the gas is helium, nitrogen, or air, and the elastic member is a spring.

22. The agent of any one of claims 16 to 21, wherein the polynucleotide is an HGF gene and/or PGIS gene.

23. The agent of any one of claims 16 to 22, wherein the oligonucleotide is an NF-κB decoy oligonucleotide comprising the sequence of SEQ ID NO: 1 or 2.

24. The agent of any one of claims 16 to 23, wherein the skin disorder is a wound, cutaneous ulcer, or psoriasis.

25. The agent of any one of claims 16 to 24, wherein the wound is a post-surgical wound or a wound caused by an injury or accident.

26. The agent of any one of claims 16 to 25, wherein the cutaneous ulcer is an intractable cutaneous ulcer.

27. The agent of any one of claims 16 to 26, wherein the intractable cutaneous ulcer is a diabetic ulcer, bedsore (pressure ulcer), or ulcer associated with venous or arterial insufficiency.

28. An agent for treating, ameliorating, or preventing a wound or cutaneous ulcer, comprising an HGF gene and/or PGIS gene as an active ingredient, wherein the agent is injected/subcutaneously introduced around diseased skin using a needleless syringe.

29. The agent of claim 28, comprising an HGF gene and a PGIS gene as active ingredients, wherein the agent is injected/subcutaneously introduced around diseased skin using a needleless syringe.

30. An agent for treating, ameliorating, or preventing psoriasis, comprising an NF-κB decoy oligonucleotide as an active ingredient, wherein the agent is injected/subcutaneously introduced around diseased skin using a needleless syringe.

31. Use of a polynucleotide for preparing an agent for treating, ameliorating, or preventing a skin disorder, wherein the agent is introduced subcutaneously using a needleless syringe.

32. Use of a polynucleotide for preparing an agent for treating, ameliorating, or preventing a skin disease, wherein the agent is injected/subcutaneously introduced around diseased skin using a needleless syringe.

33. The use of claim 31 or 32, wherein the polynucleotide is any one selected from a DNA, oligonucleotide, RNA, siRNA, and antisense.

34. The use of any one of claims 31 to 33, wherein 10 µg to 10 mg of the polynucleotide per dose is injected/subcutaneously introduced in portions to multiple sites around the diseased skin.

35. The use of any one of claims 31 to 34, wherein the needleless syringe injects the pharmaceutical liquid by using a gas pressure or an elastic force of an elastic member to drive a piston.

36. The use of claim 35, wherein the gas is helium, nitrogen, or air, and the elastic member is a spring.

37. The use of any one of claims 31 to 36, wherein the polynucleotide is an HGF gene and/or PGIS gene.

38. The use of any one of claims 31 to 37, wherein the oligonucleotide is an NF-κB decoy oligonucleotide that comprises the sequence of SEQ ID NO: 1 or 2.

39. The use of any one of claims 31 to 38, wherein the skin disorder is a wound, cutaneous ulcer, or psoriasis.

40. The use of any one of claims 31 to 39, wherein the wound is a post-surgical wound or a wound caused by an injury or accident.

41. The use of any one of claims 31 to 40, wherein the cutaneous ulcer is an intractable cutaneous ulcer.

42. The use of any one of claims 31 to 41, wherein the intractable cutaneous ulcer is a diabetic ulcer, bedsore (pressure ulcer), or ulcer associated with venous or arterial insufficiency.

43. Use of an HGF gene and/or PGIS gene for preparing an agent for treating, ameliorating, or preventing a wound or cutaneous ulcer, wherein the agent is injected/subcutaneously introduced around diseased skin using a needleless syringe.

44. The use of claim 43 of the HGF gene and PGIS gene for preparing an agent for treating, ameliorating, or preventing, wherein the agent is injected/subcutaneously introduced around diseased skin using a needleless syringe.

45. Use of an NF-κB decoy oligonucleotide for preparing an agent for treating, ameliorating, or preventing psoriasis, wherein the agent is injected/subcutaneously introduced around diseased skin using a needleless syringe.
